# EUROPEAN PATENT APPLICATION

(11) **EP 2 974 781 A1**
(43) Date of publication of application: **20.01.2016**
(21) Application number: 14176937.2
(22) Date of filing: 14.07.2014
(51) Int. Cl.: B01D 53/22, B01D 61/58, C10L 3/10

(54) **Combination of a biogas plant and a membrane filter unit for removal of carbon dioxide**

(71) Applicant: DMT Milieutechnologie B.V., 8501 SN Joure (NL)
(72) Inventor: Langerak, Jort, 8501 SN Joure (NL); Dirkse, Erwin Hendrikus Maria, 8501 SN Joure (NL)
(74) Representative: Doolaar, Frans

(57) **Abstract**

The invention relates to a combination of a biogas plant and a membrane filter unit, wherein the biogas plant produces a gas flow comprising methane and carbon dioxide, the membrane filter unit comprising:
- a first membrane filter stage with a first inlet connected to the biogas plant for receiving the gas flow, a first retentate outlet and a first permeate outlet;
- a second membrane filter stage with a second inlet connected to the first retentate outlet, a second retentate outlet and a second permeate outlet connected to the first inlet;
- a third membrane filter stage with a third inlet connected to the first permeate outlet, a third retentate outlet connected to the first inlet and a third permeate outlet; and
- a fourth membrane filter stage with a fourth inlet connected to the second retentate outlet, a fourth retentate outlet and a fourth permeate outlet.

## Description

The invention relates to a combination of a gas plant, such as a biogas plant, and a membrane filter unit, wherein the biogas plant produces a gas flow.

Biogas plants are typically used to at least partially convert biodegradable materials, such as manure, sewage, municipal waste, green waste, plant material, crops and the like, to a gas stream, which can be used for example for heating or power production. The biogas plant process is based on anaerobic digestion with anaerobic bacteria or on fermentation. Such a process produces a gas stream with mainly methane and carbon dioxide, but also some small amounts of other components. Also other gas plants, such as a small scale natural gas field or a syngas plant produce a methane rich gas containing some amounts of carbon dioxide.

The produced gas stream can be flared directly, but this involves destruction of a substantial amount of energy. The gas stream could also be used for heating or for power production with a turbine, however, the small amounts of other components typically have a high oxidizing effect, causing unacceptable wear on heating or power production devices.

Furthermore, the ratio of the amount of methane in the gas stream is typically too low to be directly fed to a gas grid transporting natural gas.

It is therefore known in the prior art to process the gas stream from the gas plant with a filter unit, typically comprising membrane filters or pressurized water scrubbing.

The advantage of membrane filters is that the gas stream can be fed to the membranes and that the carbon dioxide and the other components will permeate through the membranes, while the methane component of the gas stream will remain and two separate gas streams are obtained, one with a higher amount of methane, than the original gas stream and one with a mainly carbon dioxide component and the other components.

Such a membrane filtering step produces a gas stream with a higher content of methane and with a lower content of harmful components. Typically, one could produce by using membrane filtering a gas stream having a methane content of about 90-96%. Such a methane content is suitable for feeding the filtered gas stream to a natural gas grid.

However, feeding filtered gas, such as biogas, to a natural gas grid requires expensive control systems to ensure the quality of the fed filtered biogas.

Furthermore, the availability of the gas grid in the vicinity of the gas plant is also a requirement, which is not always that obvious, especially for smaller gas plants, such as biogas plants used by for example farmers.

Instead of transporting the gas with a natural gas grid, it is also possible to store the gas in tanks and to transport those tanks to suitable locations. However, to arrive at a cost effective way of transporting gas in tanks, the gas needs to be liquefied.

In order to liquefy gas, such as biogas, it needs to have a high methane content and more important a very low CO₂ content (of approximately 0.005%), as otherwise, the carbon dioxide portion will be solidified before the methane will be liquefied. The solid carbon dioxide will block heat exchangers and cause damage to pumps and the like, making it impossible to feed liquefied methane to a tank.

The further upgrading of the gas stream to gas qualities beyond grid quality (methane 90-96%, CO₂ 2-8%) is called polishing. Known polishing steps use cryogenic techniques or absorption techniques to arrive at the desired purity of the gas stream. However, such polishing steps are costly and are only usable in large scale biogas plants.

Accordingly, it is an object of the invention to provide a combination of gas plant, such as a biogas plant and a membrane filter unit, in which the above disadvantages are reduced or even removed.

This object is achieved with the combination according to the invention wherein the gas plant produces a gas flow comprising methane and carbon dioxide, the membrane filter unit comprising:
- a first membrane filter stage with a first inlet connected to the gas plant for receiving the gas flow, a first retentate outlet and a first permeate outlet;
- a second membrane filter stage with a second inlet connected to the first retentate outlet, a second retentate outlet and a second permeate outlet connected to the first inlet;
- a third membrane filter stage with a third inlet connected to the first permeate outlet, a third retentate outlet connected to the first inlet and a third permeate outlet; and
- a fourth membrane filter stage with a fourth inlet connected to the second retentate outlet, a fourth retentate outlet and a fourth permeate outlet.

With the combination according to the invention it has proven possible to obtain a gas flow at the fourth retentate outlet with high methane purity and a CO₂ content below 0.05%. This is obtained by the specific configuration of membrane filter stages, in which the first permeate and the second permeate are reprocessed in the membrane filter unit.

Due to the high methane, and low CO₂ content obtained with the combination according to the invention, it is possible to liquefy the gas stream of the fourth retentate outlet, such that it is easily stored in tanks and can be transported.

In a preferred embodiment of the combination according to the invention the fourth permeate outlet is connected to the first or the second inlet.

In this embodiment any methane still present in the fourth permeate outlet is recovered by the membrane filter unit to ensure that the amount of methane in the permeate stream of the membrane filter unit is kept as low as possible.

In a further embodiment of the combination according to the invention a compressor is arranged in the first inlet. The compressor ensures that the gas flow from the biogas plant is within the correct pressure range for the membrane filter stages to operate optimally.

In a further preferred embodiment of the combination according to the invention a compressor is exclusively connected to the first inlet.

It has proven that the membrane filter unit can operate optimally by using only a compressor in the first inlet and without any further compressor steps between the several membrane stages.

In yet another embodiment of the combination according to the invention pump means are connected to at least one of the permeate outlets, preferably the fourth permeate outlet.

The pump means will produce a low pressure or partial vacuum at the permeate outlet, such that the permeating action of the carbon dioxide is promoted.

Preferably, the combination according to the invention each membrane filter stage comprises a plurality of membranes, which have a selectivity for carbon dioxide over methane.

In a further embodiment of the combination according to the invention at least one of the membrane filter stages comprises a sweep gas inlet. The sweep gas provides a transport medium for transporting the permeate out of the filter stage.

In yet another preferred embodiment of the combination of the invention at least one of the membrane filter stages is arranged with temperature control means. The temperature control means preferably comprise a housing arranged around the at least one membrane filter stage and wherein the housing is provided with heating means. The heating means could comprise an electrical heating or a heat exchanger. This provides the means to control specific membrane properties like selectivity and permeability based on temperature. Therewith, broadening the applicability of the membrane module for a wider range of gas compositions and flows. Moreover, the installation operation can be easily switched between a high performance (purity) and high throughput modus without physically exchanging membrane type or numbers.

By arranging the membrane filter stage in a housing, the ambient conditions of the membrane filter stage can be controlled over the full length of the membrane, nullifying temperature effects (temperature gradient, due to expansion of gas in the permeate side) over the membrane module. Moreover, the temperature control facilitates an optimal gas quality stability, operating flexibility and control. In addition it provides means to optimize membrane quantities (Capex) and recycle streams and therefore, energy consumption (Opex).

Still a further embodiment of the combination according to the invention, further comprises liquefying means connected to the fourth or second retentate outlet.

Preferably the liquefying means liquefy the retentate by a stirling cycle or Brayton/Claude cycle.

A device performing a stirling cycle can be produced at relative low costs, while it is quite effective for liquefying methane at relatively small scale.

These and other features of the invention will be elucidated in conjunction with the accompanying drawings.
Figure 1 shows a diagram of a first embodiment of the combination according to the invention.
Figure 2 shows a diagram of a second embodiment of the combination according to the invention.

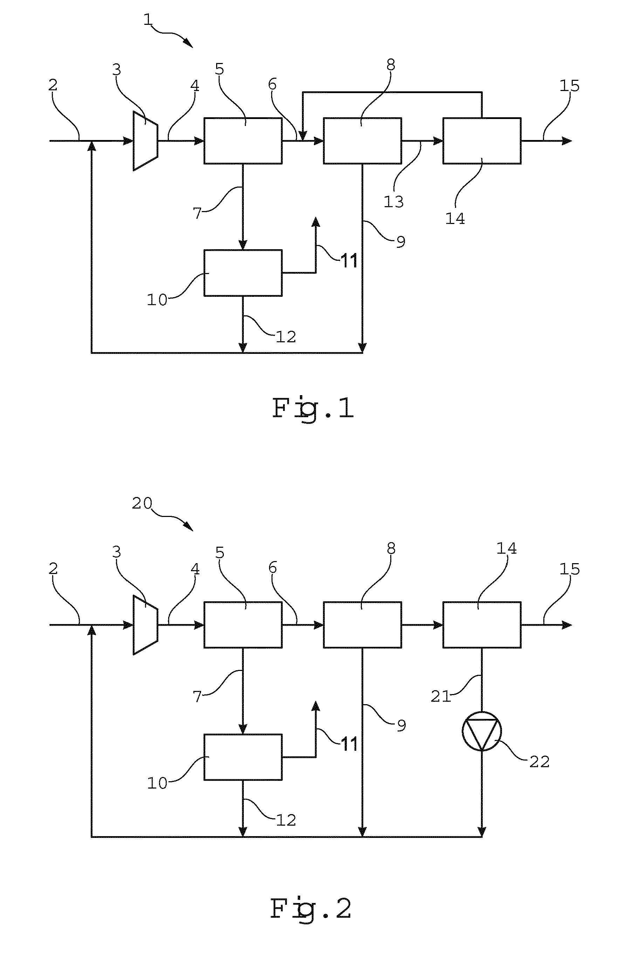

Figure 1 shows a diagram of a first embodiment 1 of the combination according to the invention. A biogas flow 2 of a biogas plant enters the membrane filter unit via a compressor 3 arranged in the first inlet 4 of a first membrane filter stage 5.

The pressurized (bio)gas, which typically is a mixture of methane and carbon dioxide enters the membranes of the membrane filter stage 5. Carbon dioxide will permeate through the membranes, while the methane will be retained within the membranes. However, the membranes will not filter all carbon dioxide, such that the first retentate outlet 6 will contain a gas stream with an increased ratio of methane versus carbon dioxide. On the other hand, some methane will permeate through the membranes, such that the first permeate outlet 7 will contain at least some methane next to a majority of carbon dioxide.

The gas flow of the first retentate outlet 6 is subsequently fed to a second membrane filter stage 8 to further filter carbon dioxide from the methane. The resulting retentate 13 containing an even higher ratio methane / carbon dioxide flows out of the second retentate outlet 9, while the permeate, containing only little methane is recirculated from the second permeate outlet 9 back to the low pressure side of the compressor 3 in the first inlet 4.

The first permeate outlet 7 of the first membrane filter stage 5 is processed by a third membrane filter stage 10, in which the major part of the carbon dioxide is vented off via the third permeate outlet 11. The remaining permeate 12, which contains mainly methane is recirculated to the low pressure side of the compressor 3 in the first inlet 4.

The second retentate outlet 13 of the second membrane filter stage 8 has already a high content of methane of about 90% or higher. In order to further filter the remaining carbon dioxide, the retentate 13 is fed to a fourth membrane filter stage 14, such that the fourth retentate outlet 15 will contain virtually only methane (carbon dioxide < 500ppm), such that the fourth retentate gas stream 15 can be fed to a liquefying device for further storage of the methane.

Any carbon dioxide filtered in the fourth filter stage 14, which still could contain some methane, will be recirculated to the third inlet 6 such that any methane is recovered.

Figure 2 shows a second embodiment 20, which corresponds largely with the first embodiment 1. Similar elements are designated with the same reference signs.

In comparison with the embodiment 1, the difference is that the fourth permeate outlet 21 is connected to a pump 22, such that a low pressure or partial vacuum is created on the permeate side of the fourth membrane filter stage 14. The low pressure assists the carbon dioxide in permeating through the membranes of the fourth membrane stage 14. As a result, the methane content in the fourth retentate outlet 15 will be further improved.

The gas flow of the fourth permeate outlet 21 is in this embodiment 20 recirculated via the pump 22 to the low pressure side of the compressor 3 in the first inlet 4.

## Claims

1. Combination of a biogas plant and a membrane filter unit, wherein the biogas plant produces a gas flow comprising methane and carbon dioxide, the membrane filter unit comprising:
- a first membrane filter stage with a first inlet connected to the biogas plant for receiving the gas flow, a first retentate outlet and a first permeate outlet;
- a second membrane filter stage with a second inlet connected to the first retentate outlet, a second retentate outlet and a second permeate outlet connected to the first inlet;
- a third membrane filter stage with a third inlet connected to the first permeate outlet, a third retentate outlet connected to the first inlet and a third permeate outlet; and
- a fourth membrane filter stage with a fourth inlet connected to the second retentate outlet, a fourth retentate outlet and a fourth permeate outlet.

2. Combination according to claim 1, wherein the fourth permeate outlet is connected to the first or the second inlet.

3. Combination according to any of the preceding claims, wherein a compressor is arranged in the first inlet.

4. Combination according to claim 3, wherein a compressor is exclusively connected to the first inlet.

5. Combination according to any of the preceding claims, wherein pump means are connected to at least one of the permeate outlets, preferably the fourth permeate outlet.

6. Combination according to any of the preceding claims, wherein each membrane filter stage comprises a plurality of membranes, which have a selectivity for carbon dioxide over methane.

7. Combination according to any of the preceding claims, wherein at least one of the membrane filter stages comprises temperature control means.

8. Combination according to claim 7, wherein the temperature control means comprise a housing arranged around the at least one membrane filter stage and wherein the housing is provided with heating means, such as an electrical heating or a heat exchanger.

9. Combination according to any of the preceding claims, further comprising liquefying means connected to the fourth or second retentate outlet.

10. Combination according to claim 9, wherein the liquefying means liquefy the retentate by a Stirling cycle or Brayton/Claude cycle.

11. Combination according to any of the preceding claims, wherein at least one of the membrane filter stages comprises a sweep gas inlet.
